# EUROPÄISCHE PATENTSCHRIFT

(11) **EP 0 680 954 B1**
(45) Veröffentlichungstag und Bekanntmachung des Hinweises auf die Patenterteilung: **22.11.2000**
(21) Anmeldenummer: 95106153.0
(22) Anmeldetag: 25.04.1995
(51) Int. Cl.: C07D 231/22

(54) **Verfahren zur Herstellung von 1-(Het)aryl-3-hydroxy-pyrazolen**
Process of preparation of 1-(Het)aryl-3-hydroxy pyrazoles
Procédé pour la préparation de 1-(Het)aryl-3-hydroxy pyrazoles

(30) Priorität: 03.05.1994 DE 4415484
(43) Veröffentlichungstag der Anmeldung: 08.11.1995
(73) Patentinhaber: BASF AKTIENGESELLSCHAFT, 67056 Ludwigshafen (DE)
(72) Erfinder: König, Hartmann, Dr., D-69115 Heidelberg (DE); Götz, Norbert, Dr., D-67547 Worms (DE); Kirstgen, Reinhard, Dr., D-67434 Neustadt (DE); Müller, Bernd, Dr., D-67227 Frankenthal (DE); Oberdorf, Klaus, Dr., D-69117 Heidelberg (DE)

(56) Entgegenhaltungen:
- EP-A- 0 366 328
- EP-A- 0 402 722
- WO-A-93/10098
- CHEMICAL &PHARMACEUTICAL BULLETIN, Bd. 19, Nr. 7, Juli 1971, TOKYO, Seiten 1389-1394, XP002024309 NORIO NAKAMURA ET AL.: "Studies on Acetylenic compounds. LVI....."
- BULLETIN DE LA SOCI T CHIMIQUE DE FRANCE, Nr. 7, Juli 1970, PARIS, Seiten 2717-2736, XP002024310 G RARD COISPEAU ET AL.: "Réaction des hydrazines avec les composés difonctionnels-1,3...."
- JOURNAL OF MEDICINAL CHEMISTRY, Bd. 19, Nr. 5, Mai 1976, COLUMBUS OHIO, Seiten 715-717, XP002024311 G. B. BENNETT ET AL.: "Synthesis and biological activity of a series of 1-Aryl-3-pyrazolidinones"

## Beschreibung

Die vorliegende Erfindung betrifft ein Verfahren zur Herstellung von 1-(Het)aryl-3-hydroxy-pyrazolen der Formel I 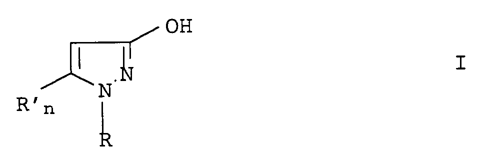 in der
- R: für einen ggf. subst. aromatischen oder heteroaromatischen Rest ausgewählt aus Phenyl, Naphthyl, 6-Ring Heteroaromaten wie Pyridin, Pyridazin, Pyrimidin, Pyrazin oder Triazin sowie 5-Ring Heteroaromaten wie Furyl, Thienyl, Pyrrolyl, Isoxazolyl, Isothiazolyl, Oxazolyl, Thiazolyl, Pyrazolyl, Imidazolyl, Oxadiazolyl, Thiadiazolyl und Triazolyl, wobei diese Reste partiell oder vollständig halogeniert sein können (d.h. die Wasserstoffatome vollständig oder teilweise durch gleiche oder verschiedene Halogenatome, insbesondere Fluor und Chlor, ersetzt sein können) und/oder eine bis drei der folgenden Gruppen tragen können: C₁-C₄-Alkyl, C₁-C₄-Halogenalkyl, C₁-C₄-Alkoxy, C₁-C₄-Halogenalkoxy und C₁-C₄-Alkylthio, und/oder wobei zwei benachbarte C-Atome dieser Reste über eine C₃-C₅-Alkylen, C₃-C₅-Halogenalkylen-, C₂-C₄-Alkylenoxy-, C₂-C₄-Halogenalkylenoxy-, C₁-C₃-Oxyalkylenoxy- oder C₁-C₃-Oxyhalogenalkylenoxykette verbrückt sein können, steht
- n: 0 oder 1 und
- R': Nitro, Cyano, Halogen, C₁-C₆-Alkyl, C₁-C₆-Halogenalkyl, C₁-C₆-Alkoxy, C₁-C₆-Alkylthio, C₃-C₆-Cycloalkyl, Phenyl, Benzyl oder C₁-C₄-Alkoxxrcarbonyl bedeutet,
aus Propiolsäurealkylestern der Formel II

R'-C≡C-CO₂R¹ II

in denen R¹ für eine Alkyl-, Cycloalkyl- oder Arylgruppe steht, und (Het)arylhydrazinen der Formel III

R-NH-NH₂ III

oder den entsprechenden Hydraziniumsalzen in einem Lösungsmittel in Gegenwart einer Base.

1-(Het)arylpyrazole können nach verschiedenen, beispielsweise in Bull. Chim. Soc. 1970, 2717, Chem. Heterocyclic Compd. (Engl. Transl.) 16, 1 (1980) oder Chem. Ber. 102, 3260 (1969) zusammengefaßten Verfahren, erhalten werden. Diese Verfahren haben aber entweder den Nachteil, daß schwer zugängliche Edukte erforderlich sind, oder daß neben den 3-Hydroxypyrazolen zu einem beträchtlichen Teil oder sogar als Hauptprodukt 5-Hydroxypyrazole gebildet werden.

Eine weitere, aus J. Med. Chem. 19, 715 (1976) bekannte Methode zur Herstellung von 3-Hydroxypyrazolen aus Acrylsäureestern und Hydrazinderivaten hat den Nachteil, daß die zunächst gebildeten Pyrazoline in einem weiteren Schritt oxidiert werden müssen, so daß die Herstellung zumindest zwei Reaktionsstufen umfaßt. Nach dem aus Chem. Pharm. Bull. 1971, 1389 bekannten Verfahren erhält man 3-Hydroxypyrazole bei der Umsetzung von Propiolsäureestern und Hydrazinen in einem Lösungsmittel in Gegenwart einer Base bei der Siedetemperatur des Lösungsmittels. Diese Verfahrensbedingungen können aber zu einer Zersetzung der Edukte führen.

Aus den in Tetrahedron Lett. 1970, 875 beschriebenen Untersuchungsergebnisen war zu erwarten, daß die Zersetzung der Edukte sich nicht durch Erniedrigung der Temperatur vermeiden ließe, da bei der Umsetzung bei Raumtemperatur im wesentlichen 5-Hydroxypyrazole gebildet wurden.

Der vorliegenden Erfindung lag daher ein einfaches und schonendes Verfahren zur Herstellung von 1-(Het)aryl-3-hydroxy-pyrazolen als Aufgabe zugrunde, das es ermöglicht auch empfindliche Edukte großtechnisch herzustellen.

Demgemäß wurde ein Verfahren zur Herstellung von 1-(Het)aryl-3-hydroxy-pyrazolen der Formel I 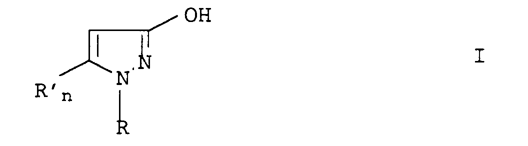 in der
R, R'und n die eingangs genannte Bedeutung haben,
aus Propiolsäureestern der Formel II

R'-C≡C-CO₂R¹ II

in denen R¹ für eine Alkyl-, Cycloalkyl- oder Arylgruppe steht, und (Het)arylhydrazinen der Formel III

R-NH-NH₂ III

in einem Lösungsmittel in Gegenwart einer Base gefunden, welches dadurch gekennzeichnet ist, daß man zunächst II und III in einem Lösungsmittel miteinander mischt und diese Mischung anschließend bei Temperaturen von 0°C bis 60°C mit der Base versetzt.

Für das erfindungsgemäße Verfahren ist es wesentlich, daß die Edukte der Formeln II und III zunächst im Lösungsmittel miteinander gemischt werden, bevor die Base - ggf. unter Kühlung - bei Temperaturen von 0°C bis 60°C, vorzugsweise 0°C bis 40°C, insbesondere 20°C bis 40°C, zugegeben wird.

Das Molverhältnis in Bezug auf die Edukte II und III ist im allgemeinen nicht kritisch. Es empfiehlt sich aber zur Vervollständigung der Umsetzung, eine der Komponenten im Überschuß einzusetzen. Demgemäß verwendet man üblicherweise 1 bis 3 mol des Propiolsäureesters II, vorzugsweise 1 bis 1,5 mol, pro mol Hydrazin III oder dem entsprechenden Hydraziniumsalz.

Die Menge an Base ist für das erfindungsgemäße Verfahren nach den bisherigen Erkenntnissen ebenfalls nicht kritisch. Üblicherweise reicht es, wenn die Base in Mengen von 1 bis 3 mol pro mol Hydrazin III oder dem entsprechenden Hydraziniumsalz, vorzugsweise 1,5 bis 2,5 mol, insbesondere 1,8 bis 2,2 mol, verwendet.

Als Lösungsmittel können grundsätzlich alle aprotischen Lösungsmittel verwendet werden, in denen sich die Edukte II und III in ausreichendem Maß lösen. So kommen beispielsweise Kohlenwasserstoffe oder halogenierte Kohlenwasserstoffe wie Dichlormethan und Tetrachlorkohlenstoff oder Ether wie Diethylether, Methyl-tert.-butylether und Tetrahydrofuran oder Gemische dieser Lösungsmittel in Betracht. Daneben kommen als Lösungsmittel auch Alkohole in Betracht, wobei der Alkohol allerdings nicht in die Reaktion eingreifen darf. Demgemäß kommen besonderes sterisch gehinderte, d.h. sekundäre oder tertiäre Alkohole, insbesondere tertiäre Alkohole wie tert.-Butanol in Betracht.

Für die Umsetzung eignen sich im allgemeinen alle nichtnucleophilen Basen. Besonders geeignet sind tertiäre Amine {z.B. N,N-Diisopropyl-N-ethylamin ("Hünig-Base"), 1,5-Diazabicyclo[4.3.0]non-5-en (DBN), 1,4-Diazabicyclo[2.2.2]octan (Dabco®) und 1,8-Diazabicyclo[5.4.0]undec-7-en (DBU)} und Alkalimetallsalze tertiärer Alkohole mit 4 bis 8 Kohenstoffatomen (z.B. Kalium-tert.-butylat).

Prinzipiell können bei der Umsetzung alle Propiolsäureester II verwendet werden, deren Esterfunktion unter den Umsetzungsbedingungen mit Hydrazin ausgetauscht werden kann. Demgemäß finden besonders Alkyl-, Cycloalkyl- oder Arylester (z.B. C₁-C₆-Alkyl, C₃-C₆-Cycloalkyl, Phenyl oder Benzyl) Verwendung. Die Art der Gruppe R' ist für die Umsetzung unbeachtlich, solange diese Gruppe nicht an der Umsetzung teilnimmt, d.h. unter den Umsetzungsbedingungen stabil ist. Als Beispiele für stabile GRuppen seien neben Wasserstoff die folgenden genannt: Nitro, Cyano, Halogen, Alkyl, Cycloalkyl, Halogenalkyl, Alkoxy, Alkylthio, Aryl oder Heteroaryl, wobei die Alkylreste üblicherweise bis zu 6 C-Atome enthalten und wobei die C-organischen Reste ihrerseits weitere unter den Umsetzungsbedingungen stabile Gruppen tragen können.

Als (Het)arylhydrazine III kommen Verbindungen in Betracht, in denen R für einen aromatischen oder heteroaromatischen Rest (z.B. Phenyl, Naphthyl, 6-Ring Heteroaromaten wie Pyridin, Pyridazin, Pyrimidin, Pyrazin oder Triazin sowie 5-Ring Heteroaromaten wie Furyl, Thienyl, Pyrrolyl, Isoxazolyl, Isothiazolyl, Oxazolyl, Thiazolyl, Pyrazolyl, Imidazolyl, Oxadiazolyl, Thiadiazolyl und Triazolyl) tragen, wobei diese Gruppen ihrerseits die vorstehend genannten unter den Umsetzungsbedingungen stabilen Gruppen tragen können.

Neben den Hydrazinen III können auch die entsprechenden Hydraziniumsalze, insbesondere die Salze von anorganischen Säuren (z.B. Halogenide wie Chloride und Bromide, Sulfate, Phosphate, Hydrogensulfate, Acetate) verwendet werden. Diese Salze verhalten sich unter den Umsetzungsverbindungen entsprechend, wenn die Menge der verwendeten Base höher bemessen wird. Demgemäß werden bei der Verwendung der Hydraziniumsalze 2 bis 4, vorzugsweise 2,5 bis 3,5, insbesondere 2,8 bis 3,2 mol einer der vorstehend genannten Basen pro mol Hydraziniumsalz verwendet.

Nach dem erfindungsgemäßen Verfahren erhält man insbesondere 1-(Het)aryl-3-hydroxypyrazole der Formel I, 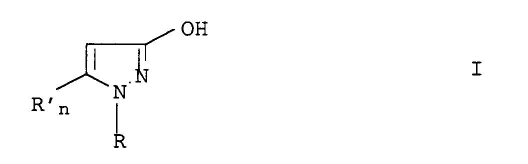 in denen die Reste und der Index die folgende Bedeutung haben:
- R: einen aromatischen oder heteroaromatischen Rest, insbesondere Phenyl, Pyridyl, Pyrimidyl, Pyrazinyl oder Pyridazinyl, wobei diese Reste partiell oder vollständig halogeniert sein können (d.h. die Wasserstoffatome vollständig oder teilweise durch gleiche oder verschiedene Halogenatome, insbesondere Fluor und Chlor, ersetzt sein können) und/oder eine bis drei der folgenden Gruppen tragen können: C₁-C₄-Alkyl, C₁-C₄-Halogenalkyl, C₁-C₄-Alkoxy, C₁-C₄-Halogenalkoxy und C₁-C₄-Alkylthio, und/oder wobei zwei benachbarte C-Atome dieser Reste über eine C₃-C₅-Alkylen-, C₃-C₅-Halogenalkylen-, C₂-C₄-Alkylenoxy-, C₂-C₄-Halogenalkylenoxy-, C₁-C₃-Oxyalkylenoxy- oder C₁-C₃-Oxyhalogenalkylenoxykette verbrückt sein können;
- n: 0, 1 oder 2, insbesondere 0 (≡ R'=Wasserstoff) oder 1, und
- R': Nitro, Cyano, Halogen (insbesondere Fluor, Chlor und Brom), C₁-C₄-Alkyl (insbesondere Methyl und Ethyl), C₁-C₄-Halogenalkyl (insbesondere Trifluormethyl, Difluormethyl und Chlormethyl), C₁-C₄-Alkoxy (insbesondere Methoxy oder Ethoxy), C₁-C₄-Halogenalkoxy (insbesondere Trifluormethoxy), C₁-C₄-Alkylthio (insbesondere Methylthio) und C₁-C₄-Alkoxycarbonyl (insbesondere Methoxycarbonyl und Ethoxycarbonyl).

Insbesondere eignet sich das erfindungsgemäße Verfahren zur Herstellung der in der folgenden Tabelle zusammengestellten 1-(Het)aryl-3-hydroxypyrazole I, die zum Teil aus der Literatur bekannt und zum Teil neu sind.

| **R'** | **R** | | **R'** | **R** |
|---|---|---|---|---|
| H | C₆H₅ | | H | 2,5-Cl₂-C₆H₃ |
| H | 4-CH₃-C₆H₄ | | H | 3,4-Cl₂-C₆H₃ |
| H | 3-Cl-C₆H₄ | | H | 3-CF₃-C₆H₄ |
| H | 4-Cl-C₆H₄ | | H | 5-CF₃-C₆H₄ |
| H | 2,4-(CH₃)₂-C₆H₃ | | H | 3-OCH₃-C₆H₄ |
| H | 2-CH₃, 4-Cl-C₆H₃ | | H | 3,4-[OCF₂O]-C₆H₃ |
| H | 2,6-Cl₂-C₆H₃ | | H | 4-CF₃-C₆H₄ |
| H | 2-CH₃-C₆H₄ | | H | pyridin-2-yl |
| H | 3-CH₃-C₆H₄ | | 5-CH₃ | C₆H₅ |
| H | 4-OCH₃-C₆H₄ | | 5-CF₃ | 2,4-Cl₂-C₆H₃ |
| H | 5-Cl-pyridin-2-yl | | H | 4-OCF₃-C₆H₄ |
| H | 1-pyrazinyl | | H | 3-Cl-6-pyridazinyl |

Die nach dem erfindungsgemäßen Verfahren erhältlichen Verbindungen I eignen sich als Zwischenprodukte bei der Herstellung von Farb- oder Wirkstoffen. Von besonderer Bedeutung sind sie für die Synthese von Wirkstoffen der Formel 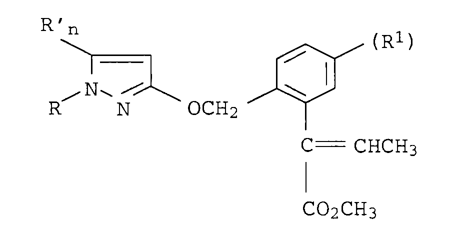 die in der zeitgleichen Anmeldung DE Anm. Nr. 44 15 483 für die Bekämpfung von Schadpilzen und tierischen Schädlingen beschrieben sind.

### Verfahrensbeispiele:

1. 3-Hydroxy-1-(4-methoxyphenyl)-pyrazol
   Eine Lösung von 72,7 g 4-Methoxyphenylhydrazin in 700 ml tert.-Butanol wurde bei 30°C tropfenweise mit 56,8 g Propiolsäureethylester versetzt. In die so erhaltene Mischung wurde anschließend unter Eiskühlung portionsweise insgesamt 118 g Kalium-tert.-butylat eingetragen. Nach 12 h bei 25°C wurde das Lösungsmittel bei vermindertem Druck abdestilliert und der so erhaltene Rückstand wurde in Wasser aufgenommen. Die wäßrige Lösung wurde mit Dichlormethan gereinigt und anschließend unter Kühlung mit konz. Essigsäure bis zu einem pH-Wert von 9 angesäuert, wobei das Produkt als Feststoff ausfiel. Nach Waschen und Trocknen erhielt man 60,3 g der Titelverbindung (60% der Theorie); Fp.: 167-170°C.
2. 3-Hydroxy-1-(4-trifluoromethoxyphenyl)-pyrazol
   Gemäß der unter 1. beschriebenen Verfahrensweise erhielt man aus 8,4 g 4-Trifluoromethoxyphenylhydrazin und 4,7 g Propiolsäureethylester (100 ml tert.-Butanol, 9,9 g Kalium-tert.-butylat) 2,43 g der Titelverbindung (23% der Theorie); ¹NMR (DMSO/TMS): 10,40 (s-br, 1H), 8,35 (d, 1H), 7,80 (d, 2H), 7,45 (d, 2H), 5,90 (d, 1H).
3. 3-Hydroxy-1-(5-trifluoromethylpyridin-2-yl)-pyrazol
   Gemäß der unter 1. beschriebenen Verfahrensweise erhielt man aus 76,1 g 5-Trifluoromethylpyridin-2-ylhydrazin und 46,3 g Propiolsäureethylester (700 ml tert.-Butanol, 96,3 g Kalium-tert.-butylat) 57,2 g der Titelverbindung (58% der Theorie); Fp.: 199-205°C.
   Gemäß der in Chem. Pharm. Bull. 19, 1394 (1971) in Beispiel XXI gegebenen Vorschrift konnte bei der Umsetzung von 8,9 g 5-Trifluoromethylpyridin-2-ylhydrazin und 8,9 g Propiolsäuremethylester (100 ml tert.-Butanol, 11,2 g Kalium-tert.-butylat) kein Produkt nachgewiesen werden.
4. 3-Hydroxy-1-(2-pyridyl)-pyrazol
   Gemäß der unter 1. beschriebenen Verfahrensweise erhielt man aus 32,7 g Pyridin-2-ylhydrazin und 64,7 g Propiolsäureethylester (600 ml tert.-Butanol, 134,4 g Kalium-tert.-butylat) 19,1 g der Titelverbindung (40% der Theorie); ¹NMR (DMSO/TMS): 10,40 (s-br, 1H), 8,40 (m, 2H), 7,90 (m, 1H), 7,70 (d, 1H), 7,20 (m, 1H), 5,90 (d, 1H).

## Patentansprüche

1. Verfahren zur Herstellung von 1-(Het)aryl-3-hydroxy-pyrazolen der Formel I 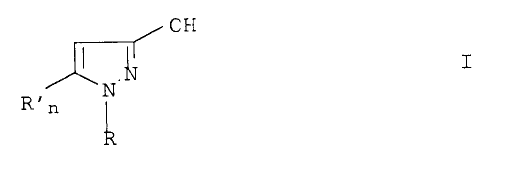 in der
R für einen ggf. subst. aromatischen oder heteroaromatischen Rest ausgewählt aus Phenyl, Naphthyl, 6-Ring Heteroaromaten wie Pyridin, Pyridazin, Pyrimidin, Pyrazin oder Triazin sowie 5-Ring Heteroaromaten wie Furyl, Thienyl, Pyrrolyl, Isoxazolyl, Isothiazolyl, Oxazolyl, Thiazolyl, Pyrazolyl, Imidazolyl, Oxadiazolyl, Thiadiazolyl und Triazolyl, wobei diese Reste partiell oder vollständig halogeniert sein können (d.h. die Wasserstoffatome vollständig oder teilweise durch gleiche oder verschiedene Halogenatome, insbesondere Fluor und Chlor, ersetzt sein können) und/oder eine bis drei der folgenden Gruppen tragen können: C₁-C₄-Alkyl, C₁-C₄-Halogenalkyl, C₁-C₄-Alkoxy, C₁-C₄-Halogenalkoxy und C₁-C₄-Alkylthio, und/oder wobei zwei benachbarte C-Atome dieser Reste über eine C₃-C₅-Alkylen-, C₃-C₅-Halogenalkylen-, C₂-C₄-Alkylenoxy-, C₂-C₄-Halogenalkylenoxy-, C₁-C₃-Oxyalkylenoxy- oder C₁-C₃-Oxyhalogenalkylenoxykette verbrückt sein können, steht
n 0 oder 1 und
R' Nitro, Cyano, Halogen, C₁-C₆-Alkyl, C₁-C₆-Halogenalkyl, C₁-C₆-Alkoxy, C₁-C₆-Alkylthio, C₃-C₆-Cycloalkyl, Phenyl, Benzyl oder C₁-C₄-Alkoxycarbonyl bedeutet,
aus Propiolsäureestern der Formel II
R'-C≡C-CO₂R¹ II
in denen R¹ für eine Alkyl-, Cycloalkyl- oder Arylgruppe steht, und (Het)arylhydrazinen der Formel III
R-NH-NH₂ III
oder den entsprechenden Hydraziniumsalzen in einem Lösungsmittel in Gegenwart einer Base, dadurch gekennzeichnet, daß man zunächst II und III in einem Lösungsmittel miteinander mischt und diese Mischung anschließend bei Temperaturen von 0°C bis 60°C mit der Base versetzt.

2. Verfahren nach Anspruch 1, dadurch gekennzeichnet, daß man als Base ein tertiäres Amin verwendet.

3. Verfahren nach Anspruch 1, dadurch gekennzeichnet, daß man als Base ein Alkalimetallsalz eines Alkohols mit 4 bis 8 Kohlenstoffatomen verwendet.

4. Verfahren nach Anspruch 1, dadurch gekennzeichnet, daß man die Umsetzung in einem aprotischen Lösungsmittel durchführt.

5. Verfahren nach Anspruch 1, dadurch gekennzeichnet, daß man die Umsetzung in einem tertiären Alkohol durchführt.

6. Verfahren nach Anspruch 1, dadurch gekennzeichnet, daß man die Base in einer Menge von 1 bis 3 mol pro mol III verwendet.

7. Verfahren nach Anspruch 1, dadurch gekennzeichnet, daß man pro mol der Verbindung III 1 bis 3 mol der Verbindung II verwendet.

8. Verfahren nach Anspruch 1, dadurch gekennzeichnet, daß man anstelle der (Het)arylhydrazine III ein entsprechendes Hydraziniumsalz verwendet.

## Claims

1. A process for preparing 1-(het)aryl-3-hydroxypyrazoles of the formula I 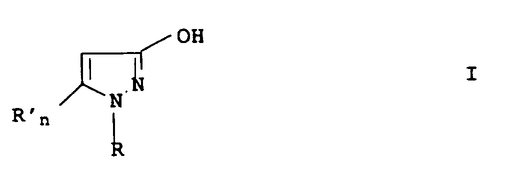 where
R is an unsubst. or subst. aromatic or heteroaromatic radical selected from phenyl, naphthyl, 6-membered ring heteroaromatics such as pyridine, pyridazine, pyrimidine, pyrazine or triazine, and 5-membered ring heteroaromatics such as furyl, thienyl, pyrrolyl, isoxazolyl, isothiazolyl, oxazolyl, thiazolyl, pyrazolyl, imidazolyl, oxadiazolyl, thiadiazolyl and triazolyl, it being possible for these radicals to be partially or completely halogenated (ie. it being possible for the hydrogen atoms to be replaced completely or partly by identical or different halogen atoms, in particular fluorine and chlorine) and/or to carry one to three of the following groups: C₁-C₄-alkyl, C₁-C₄-haloalkyl, C₁-C₄-alkoxy, C₁-C₄-haloalkoxy and C₁-C₄-alkylthio, and/or it being possible for two adjacent C atoms of these radicals to be bridged via a C₃-C₅-alkylene, C₃-C₅-haloalkylene, C₂-C₄-alkylenoxy, C₂-C₄-haloalkylenoxy, C₁-C₃-oxyalkylenoxy or C₁-C₃-oxyhaloalkylenoxy chain,
n is 0 or 1 and
R' is nitro, cyano, halogen, C₁-C₆-alkyl, C₁-C₆-haloalkyl, C₁-C₆-alkoxy, C₁-C₆-alkylthio, C₃-C₆-cycloalkyl, phenyl, benzyl or C₁-C₄-alkoxycarbonyl,
from propiolic acid esters of the formula II
R'-C≡C-CO₂R¹ II
where R¹ is an alkyl, cycloalkyl or aryl group, and (het)arylhydrazines of the formula III
R-NH-NH₂ III
or the corresponding hydrazinium salts in a solvent in the presence of a base, which comprises first mixing II and III with one another in a solvent and then treating this mixture with the base at from 0°C to 60°C.

2. A process as claimed in claim 1, wherein the base used is a tertiary amine.

3. A process as claimed in claim 1, wherein the base used is an alkali metal salt of an alcohol having 4 to 8 carbon atoms.

4. A process as claimed in claim 1, wherein the reaction is carried out in an aprotic solvent.

5. A process as claimed in claim 1, wherein the reaction is carried out in a tertiary alcohol.

6. A process as claimed in claim 1, wherein the base is used in an amount from 1 to 3 mol per mole of III.

7. A process as claimed in claim 1, wherein, per mole of the compound III, 1 to 3 mol of the compound II are used.

8. A process as claimed in claim 1, wherein, instead of the (het)arylhydrazines III, an appropriate hydrazinium salt is used.

## Revendications

1. Procédé pour la préparation de 1-(hét)aryl-3-hydroxy-pyrazoles de formule I 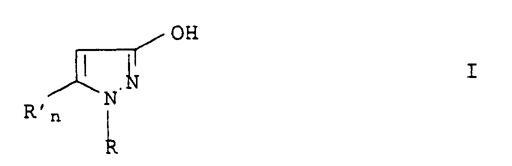 où
R désigne un reste aromatique ou hétéroaromatique éventuellement substitué, choisi parmi les restes phényle, naphtyle, hétéroaromatiques à 6 chaînons tels que pyridine, pyridazine, pyrimidine, pyrazine ou triazine, ainsi que hétéroaromatiques à 5 chaînons tels que furyle, thiényle, pyrrolyle, isoxazolyle, isothiazolyle, oxazolyle, thiazolyle, pyrazolyle, imidazolyle, oxadiazolyle, thiadiazolyle et triazolyle, tandis que ces restes peuvent être partiellement ou totalement halogénés (c'est-à-dire que les atomes d'hydrogène peuvent être remplacés en totalité ou en partie par des atomes d'halogène identiques ou différents, en particulier fluor et chlore) et/ou peuvent porter un à trois des groupes ci-après: alkyle en C₁-C₄, halogénoalkyle en C₁-C₄, alcoxy en C₁-C₄, halogénoalcoxy en C₁-C₄ et alkyl(C₁-C₄)thio, et/ou tandis que deux atomes de carbone contigus de ces restes peuvent être reliés par une chaîne alkylène en C₃-C₅, halogénoalkylène en C₃-C₅, alkylène(C₂-C₄)oxy, halogénoalkylène(C₂-C₄)oxy, oxyalkylène(C₁-C₃)oxy ou oxyhalogénoalkylène(C₁-C₃)oxy,
n vaut 0 ou 1 et
R' représente un groupe nitro, cyano, halogéno, alkyle en C₁-C₆, halogénoalkyle en C₁-C₆, alcoxy en C₁-C₆, alkyl(C₁-C₆)thio, cycloalkyle en C₃-C₆, phényle, benzyle ou alcoxy(C₁-C₄)carbonyle,
à partir d'esters d'acide propiolique de formule II
R'-C≡C-CO₂R¹ II
dans lesquels R¹ désigne un groupe alkyle, cycloalkyle ou aryle, et de (hét)arylhydrazines de formule III
R-NH-NH₂ III
ou des sels d'hydrazinium correspondants dans un solvant en présence d'une base, caractérisé par le fait qu'on mélange d'abord entre eux II et III dans un solvant, et on ajoute ensuite la base à ce mélange à des températures de 0°C à 60°C.

2. Procédé selon la revendication 1, caractérisé par le fait qu'on utilise comme base une amine tertiaire.

3. Procédé selon la revendication 1, caractérisé par le fait qu'on utilise comme base un sel de métal alcalin d'un alcool ayant 4 à 8 atomes de carbone.

4. Procédé selon la revendication 1, caractérisé par le fait qu'on effectue la réaction dans un solvant aprotique.

5. Procédé selon la revendication 1, caractérisé par le fait qu'on effectue la réaction dans un alcool tertiaire.

6. Procédé selon la revendication 1, caractérisé par le fait qu'on utilise la base en une quantité de 1 à 3 mol par mol de III.

7. Procédé selon la revendication 1, caractérisé par le fait qu'on utilise par mol du composé III 1 à 3 mol du composé II.

8. Procédé selon la revendication 1, caractérisé par le fait qu'on utilise à la place de la (hét)arylhydrazine III un sel d'hydrazinium correspondant.
